# EUROPEAN PATENT APPLICATION

(11) **EP 2 829 869 A1**
(43) Date of publication of application: **28.01.2015**
(21) Application number: 14167058.8
(22) Date of filing: 05.05.2014
(51) Int. Cl.: G01N 27/414, G01N 27/416

(54) **Apparatus and method for compensating pH measurement errors due to pressure and physical stresses**

(30) Priority: 22.07.2013 US 201313947924
(71) Applicant: Honeywell International Inc., Morristown, NJ 07962-2245 (US)
(72) Inventor: Horkheimer, Donald, Morristown NJ New Jersey 07962-2245 (US); Willits, David S., Morristown NJ New Jersey 07962-2245 (US)
(74) Representative: Houghton, Mark Phillip

(57) **Abstract**

A pH sensing apparatus includes an ion-sensing cell that includes a first half-cell including a first Ion-Sensitive Field Effect Transistor (ISFET) exposed to a surrounding solution; and a second reference half-cell exposed to the surrounding solution. The pH sensing apparatus further includes a pressure sensitivity compensation loop including a Non Ion-Sensitive Field Effect Transistor (NISFET). The pH sensing apparatus is configured to compensate for at least one of pressure and physical stresses using signals from the ion-sensing cell and feedback from the pressure sensitivity compensation loop. The pH sensing cell further includes a processing device configured to calculate a final pH reading compensated to minimize the at least one of pressure and physical stresses.

## Description

### STATEMENT REGARDING FEDERALLY

### SPONSERED RESEARCH OR DEVELOPMENT

This invention was made with Government support under N00014-10-1-0206 awarded by Office of Naval Research. The Government has certain rights in the invention.

### BACKGROUND

Researchers measure CO₂ levels in the ocean to monitor global warming risks and ocean health. Measuring ocean pH at various depths is one method researchers use to determine CO₂ levels in the ocean.

### SUMMARY

A pH sensing apparatus includes an ion-sensing cell that includes a first half-cell including a first Ion-Sensitive Field Effect Transistor (ISFET) exposed to a surrounding solution; and a second reference half-cell exposed to the surrounding solution. The pH sensing apparatus further includes a pressure sensitivity compensation loop including a Non Ion-Sensitive Field Effect Transistor (NISFET). The pH sensing apparatus is configured to compensate for at least one of pressure and physical stresses using signals from the ion-sensing cell and feedback from the pressure sensitivity compensation loop. The pH sensing cell further includes a processing device configured to calculate a final pH reading compensated to minimize the at least one of pressure and physical stresses.

### DRAWINGS

Understanding that the drawings depict only exemplary embodiments and are not therefore to be considered limiting in scope, the exemplary embodiments will be described with additional specificity and detail through the use of the accompanying drawings, in which:
Figure 1A-1D show block diagrams of exemplary pH sensing apparatuses.
Figure 2A shows a more detailed schematic of an exemplary embodiment of an exemplary pH sensing apparatus of Figure 1A.
Figure 2B shows a more detailed schematic of an exemplary embodiment of an exemplary pH sensing apparatus of Figure 1A.
Figure 3A shows a more detailed schematic of an exemplary embodiment of an exemplary pH sensing apparatus of Figure 1B.
Figure 3B shows a more detailed schematic of an exemplary embodiment of an exemplary pH sensing apparatus of Figure 1B.
Figure 4A shows a more detailed schematic of an exemplary embodiment of an exemplary pH sensing apparatus of Figure 1C.
Figure 4B shows a more detailed schematic of an exemplary embodiment of an exemplary pH sensing apparatus of Figure 1C.
Figure 5A shows a more detailed schematic of an exemplary embodiment of an exemplary pH sensing apparatus of Figure 1D.
Figure 5B shows a more detailed schematic of an exemplary embodiment of an exemplary pH sensing apparatus of Figure 1D.
Figure 6 shows an exemplary method of limiting measurement error for an output of a pH sensor.

In accordance with common practice, the various described features are not drawn to scale but are drawn to emphasize specific features relevant to the exemplary embodiments.

### DETAILED DESCRIPTION

In the following detailed description, reference is made to the accompanying drawings that form a part hereof, and in which is shown by way of illustration specific illustrative embodiments. However, it is to be understood that other embodiments may be utilized and that logical, mechanical, and electrical changes may be made. Furthermore, the method presented in the drawing figures and the specification is not to be construed as limiting the order in which the individual steps may be performed. The following detailed description is, therefore, not to be taken in a limiting sense.

One formation of a pH sensor is a solid-state semiconductor device known as an Ion-Sensitive Field Effect Transistor (ISFET). In exemplary embodiments, ISFETs are used in combination with reference half-cells to measure the pH of a surrounding solution. Current pH sensing device accuracy is limited by measurement error induced by large mechanical stresses associated with use in deep seas and packaging stresses associated with making the sensor strong enough to operate over a wide pressure variation and cyclic loading over a long period of time. Additionally, the accuracy of pH sensors is limited due to the temperature sensitivity of ISFETs. There is a demand for a deep sea pH sensor that is resistant to these stresses.

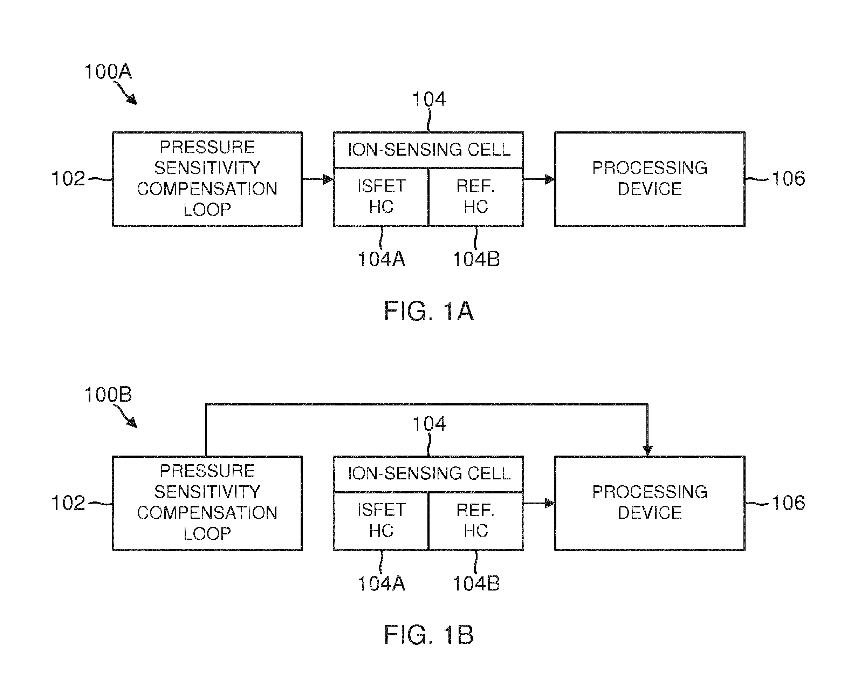

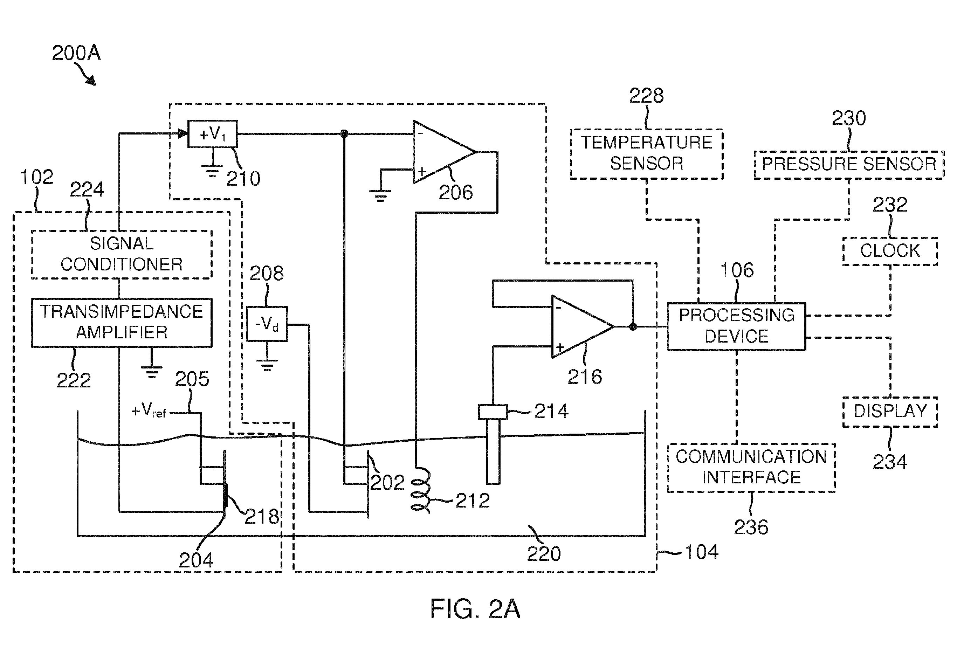

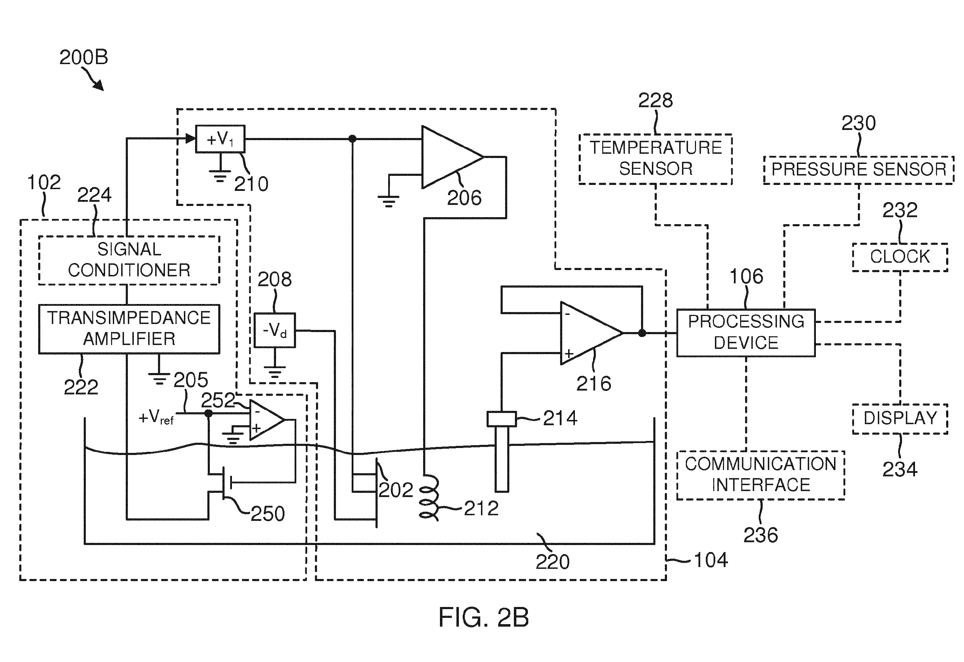

Figure 1A is a block diagram of an exemplary pH sensing apparatus 100A. The apparatus 100A includes three main components: a pressure sensitivity compensation loop 102, an ion-sensing cell 104, and a processing device 106. The ion-sensing cell 104 is subdivided into two half-cells: an ISFET half-cell 104A and a reference half-cell 104B. The ISFET half-cell 104A includes a first ISFET (such as first ISFET 202 shown in Figures 2-5 below). In other exemplary embodiments, the ion-sensing cell 104 is not divided into two half-cells. In other exemplary embodiments, the ion-sensing cell 104 is divided into greater quantities of sub-cells.

In exemplary embodiments of pH sensing apparatus 100A, the pressure sensitivity compensation loop 102 includes a Non Ion-Sensitive Field Effect Transistor (NISFET) (such as NISFET 204 or NISFET 250). In exemplary embodiments, the NISFET (such as NISFET 204) is an ISFET that has been sealed with an ion-blocking film (such as ion-blocking film 218), such that it is no longer sensitive to the ions in a solution under test (such as a solution under test 220) such as sea water. An exemplary ion-blocking film (such as ion-blocking film 218) comprises a metal deposition to disable the gate and an insulative deposition to prevent the metal deposition from corroding. In exemplary embodiments, the metal deposition comprises gold, platinum, titanium, tantalum, nickel, chromium, aluminum, tungsten, iridium, or silver. In exemplary embodiments, the insulative deposition comprises silicon oxide, aluminum oxide, diamond like carbon (DLC), aluminum nitride, glass compositions, tantalum oxide, beryllium oxide, or silicon nitride. In exemplary embodiments, the NISFET (such as NISFET 250) is a Metal-Oxide-Semiconductor Field Effect Transistor. In exemplary embodiments, the NISFET (such as NISFET 204 or NISFET 250) has substantially equivalent pressure and temperature sensitivities as the first ISFET (such as first ISFET 202). The closer the pressure and temperature sensitivities of the NISFET (such as NISFET 204 or NISFET 250) are to the first ISFET (such as first ISFET 202), the better the differential setup is able to provide compensation benefits. In exemplary embodiments, the ISFET (such as ISFET 202) and NISFET (such as NISFET 204 or NISFET 250) will have a common silicon substrate. In exemplary embodiments, the ISFET (such as ISFET 202) and NISFET (such as NISFET 204 or NISFET 250) are fabricated on a common wafer.

The pressure sensitivity compensation loop 102 of the exemplary pH sensing apparatus 100A provides analog feedback directly to the ISFET half-cell 104A, which compensates the potential of the ISFET half-cell 104A for at least one of pressure and physical stresses. In exemplary embodiments, the processing device is configured to receive the potential of the reference half-cell 104B and further determine the pH level from the difference in potentials of the ISFET half-cell 104A and the reference half-cell 104B.

Figure 1B is a block diagram of an exemplary pH sensing apparatus 100B. In the exemplary pH sensing apparatus 100B, the feedback from the pressure sensitivity compensation loop 102 does not go to the ISFET half-cell 104A directly. Instead, the pressure sensitivity compensation loop 102 provides digital feedback to the processing device 106. The processing device 106 then compensates the measured pH level from the ion-sensing cell 104 for at least one of pressure and physical stresses using the feedback from the pressure sensitivity compensation loop 102.

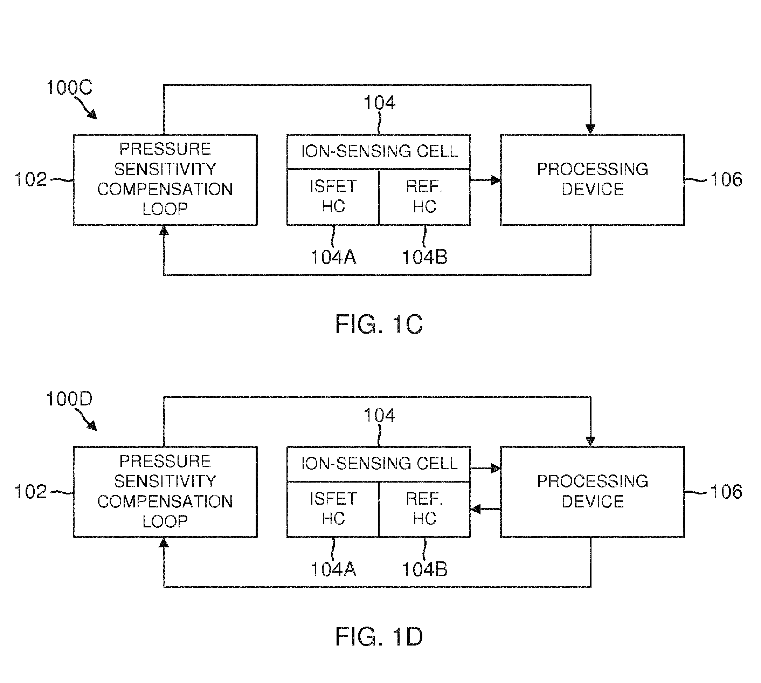

Figure 1C is a block diagram of an exemplary pH sensing apparatus 100C. As with exemplary pH sensing apparatus 100B, the pressure sensitivity compensation loop 102 of the exemplary pH sensing apparatus 100C provides digital feedback to the processing device 106. The processing device 106 is communicatively coupled to the source of the NISFET (such as NISFET 204 or NISFET 250) and provides a voltage. In exemplary embodiments, the processing device 106 also provides a voltage to gate of the NISFET (such as NISFET 250) of the pressure sensitivity compensation loop 102. The processing device 106 then compensates the measured pH level from the ion-sensing cell 104 for at least one of pressure and physical stresses using the feedback from the pressure sensitivity compensation loop 102 and by adjusting the voltage(s) provided to the NISFET. By allowing the processing device 106 to directly control the NISFET, the compensation performed by the processing device 106 for at least one of pressure and physical stresses is more sophisticated than in the exemplary pH sensing apparatus 100B.

Figure 1D is a block diagram of an exemplary pH sensing apparatus 100D. As with exemplary pH sensing apparatus 100B and exemplary pH sensing apparatus 100C, the pressure sensitivity compensation loop 102 of the exemplary pH sensing apparatus 100D provides digital feedback to the processing device 106. The processing device 106 is communicatively coupled to the source of the NISFET (such as NISFET 204 or NISFET 250) and provides a voltage. In exemplary embodiments, the processing device 106 also provides a voltage to the NISFET (such as NISFET 204) of the pressure sensitivity compensation loop 102, similar to exemplary pH sensing apparatus 100C. The processing device 106 also provides a voltage to the ISFET half-cell 104A of the ion-sensing cell 104. The processing device 106 then compensates the measured pH level from the ion-sensing cell 104 for at least one of pressure and physical stresses using the feedback from the pressure sensitivity compensation loop 102, by adjusting the voltage(s) provided to the NISFET, and by adjusting the voltage provided to the ISFET half-cell 104A of the ion-sensing cell 104. By allowing the processing device 106 to directly control the ISFET, the compensation performed by the processing device 106 for at least one of pressure and physical stresses is more sophisticated than in the exemplary pH sensing apparatus 100C.

Figure 2A shows an embodiment of a pH sensing apparatus 200A in accordance with the present invention. The pH sensing apparatus 200A includes a first ISFET 202 and a NISFET 204, where the ISFET 202 and NISFET 204 are in a differential setup. The source of the first ISFET 202 is communicatively coupled to the inverting input of an amplifier 206. A voltage source 208 (-V_{d}) controls the drain-source voltage of the ISFET 202 at a preselected level. A voltage source 210 (+V₁) drives the ISFET 202 and the amplifier 206. A reference electrode 214 is communicatively coupled to the input of an amplifier 216. In an exemplary embodiment, the reference electrode 214 comprises Ag/Ag Halides (such as Ag/AgCl, Ag/AgI, and Ag/AgBr), Hg/HgO, Hg/Hg Halides, Ir/IrO₂, or Rare Earth Halides. In other exemplary embodiments, the reference electrode 214 is replaced by a Reference Field Effect Transistor (REFET) and a quasi-reference electrode. In the exemplary embodiment of the pH sensing apparatus 200A shown in Figure 2A, the output of the amplifier 206 drives a counter electrode 212 so as to keep the source of the first ISFET 202 at circuit common. Circuit common is the potential at the non-inverting input of the amplifier 206. In exemplary embodiments, the counter electrode 212 comprises a metal wire, a metallic portion of the pH sensor housing, or another conductive metal surface in contact with the solution under test. The counter electrode 112 also reduces spurious currents on the reference electrode 214. In other embodiments, a counter electrode 212 is not included.

In Figure 2A, the drain current of the NISFET 204, which is shown as a sealed ISFET, is controlled by a source of potential 205 (+V_{ref}). The drain of the NISFET 204 is communicatively coupled to a transimpedance amplifier 222, which is communicatively coupled to the voltage source 210. A signal conditioner 224 could optionally be added to this connection to reduce noise.

The output of the amplifier 216 is communicatively coupled to a processing device 106. Figure 2A also includes additional inputs (including sensors and clocks) and outputs (including communication interfaces and displays) that can be optionally communicatively coupled to the processing device 106 for use in sensor compensation and transmission and/or display. In exemplary embodiments, the additional inputs include: at least one temperature sensor 228, at least one pressure sensor 230, at least one reference clock 232 (such as a Global Navigation Satellite System (GNSS) based clock), a display 234, and/or a communication interface 236. In exemplary embodiments, the at least one temperature sensor 228, at least one pressure sensor 230, and at least one reference clock 232 are used by the processing device 106 to perform additional compensations. In exemplary embodiments, the at least one temperatures sensor 228 is configured to measure temperature at a point in the pH sensing apparatus 200A which can then be used to further compensate the output. In exemplary embodiments, the at least one pressure sensor is configured to measure pressure at a point in the pH sensing apparatus which can then be used to further compensate the output of the pH sensing apparatus 200A.

In exemplary embodiments where more than one temperature sensor 228 is used, a thermal gradient may be measured and compensated for. In exemplary implementations, the temperature at a plurality of points in the apparatus is measured with a plurality of temperature sensors (such as sensor 228) and a known distance between the plurality of temperature sensors are used to calculate the thermal gradient. In exemplary implementations, the temperature is measured at substantially the same time. In exemplary implementations, the temperature sensors are synchronized using the reference clock 232 such that the plurality of temperatures sensors measure temperature at substantially the same time. In exemplary embodiments, the processing device is further configured to determine the thermal gradient between the plurality of points based on a difference in temperature at the plurality of points in the apparatus and the known distance between the plurality of temperature sensors. In exemplary implementations, the gradient is calculated by dividing the change in temperature between the plurality of temperature sensors by the distance between the sensors.

In exemplary embodiments, the display 234 displays the compensated pH reading or other information. In exemplary embodiments, the communication interface 236 is used to communicate the compensated pH reading or other information to another device, another system, and/or another apparatus. In exemplary embodiments, the communication interface 236 includes at least one of a wired communication port and a wireless communication transceiver and antenna.

The variation in the voltage output of the NISFET 204 is related to the pressure and physical stresses experienced by it. Since the NISFET 204 has the same pressure and temperature sensitivities as the ISFET 202, the pressure and physical stresses experienced by both should be the same. By providing analog feedback from the NISFET 204 to trim the voltage source 210 that is driving the ISFET 202 and amplifier 206, the variation in the voltage output of the ISFET 202 due to at least one of pressure and physical stresses can be compensated for. This compensation will result in a more accurate pH reading than can be achieved without using feedback from the NISFET 204.

In exemplary embodiments, the pH sensing apparatus 200A is a specific implementation of the exemplary embodiment of pH sensing apparatus 100A shown in Figure 1A and includes a pressure sensitivity compensation loop 102, an ion-sensing cell 104, and the processing device 106 as shown in Figure 2A. In exemplary embodiments, the ion-sensing cell 104 of pH sensing apparatus 200A includes the first ISFET 202, the reference electrode 214, the counter electrode 212, the amplifier 206, the amplifier 216, the voltage source 208, and the voltage source 210. In exemplary embodiments, the pressure sensitivity compensation loop 102 of pH sensing apparatus 200A includes the NISFET 204, the source of potential 205, the transimpedance amplifier 222, and the optional signal conditioner 224.

As mentioned above, in exemplary embodiments, a NISFET is a sealed ISFET or a MOSFET. Figure 2B shows an embodiment of a pH sensing apparatus 200B in accordance with the present invention. The pH sensing apparatus 200B is the same as pH sensing apparatus 200A, except that the NISFET 204 of Figure 2A is replaced with a NISFET 250, which is a MOSFET. In exemplary embodiments, the size of the channel in the NISFET 250 is controlled by the output of an amplifier 252 and the source of potential 205 (+V_{ref}). The same compensation capabilities from using the differential setup of pH sensing apparatus 200A are available using the differential setup of pH sensing apparatus 200B.

In exemplary embodiments, the pH sensing apparatus 200B is a specific implementation of the exemplary embodiment of pH sensing apparatus 100A shown in Figure 1A and includes a pressure sensitivity compensation loop 102, an ion-sensing cell 104, and the processing device 106 as shown in Figure 2B. In exemplary embodiments, the ion-sensing cell 104 of pH sensing apparatus 200B includes the first ISFET 202, the reference electrode 214, the counter electrode 212, the amplifier 206, the amplifier 216, the voltage source 208, and the voltage source 210. In exemplary embodiments, the pressure sensitivity compensation loop 102 of pH sensing apparatus 200A includes the NISFET 250, the source of potential 205, the amplifier 252, the transimpedance amplifier 222, and the optional signal conditioner 224.

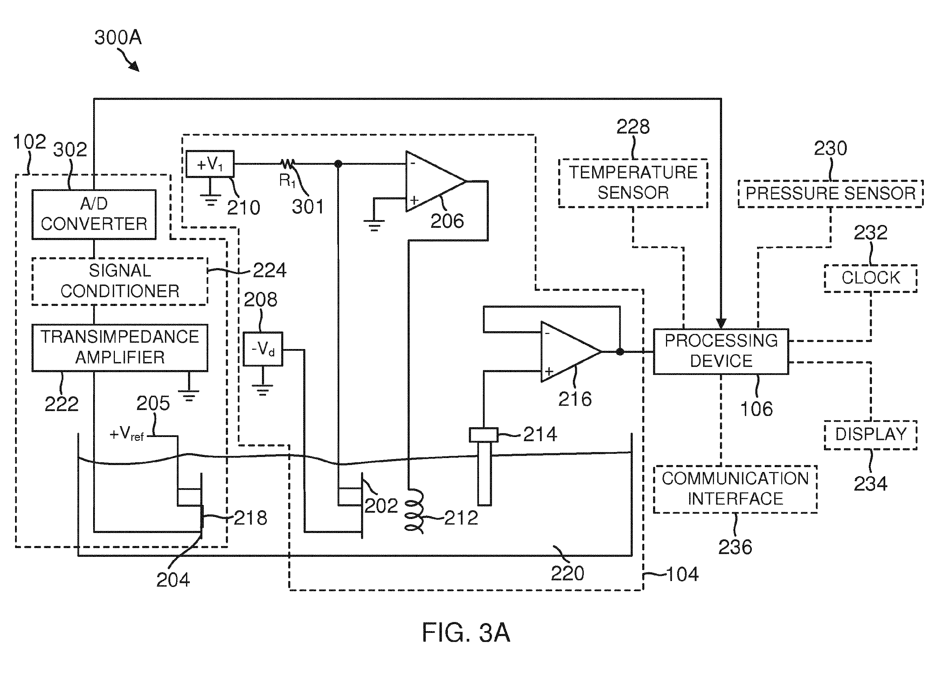

Figure 3A shows another embodiment of a pH sensing apparatus 300A in accordance with the present invention. This embodiment is similar to apparatus 200A of Figure 2A, so only the differences in the circuit will be discussed.

In the apparatus 300A, the drain of the NISFET 204 is not communicatively coupled to the voltage source 210. In exemplary embodiments, due to this change from the exemplary pH sensing apparatus 200A of Figure 2A, a resistor 301 (R₁) is added to the circuit to control the drain-source current of the ISFET 202. The drain of the NISFET 204 is communicatively coupled to a processing device 106 through a transimpedance amplifier 220 and an analog-to-digital converter 302. A signal conditioner 224 could optionally be added to reduce noise on the signal. The variation in the voltage output of the NISFET 204 is related to the pressure and physical stresses experienced by it. By converting the output of the NISFET 204 to a digital signal and sending the signal to the processing device 106, the variation in the voltage output of the ISFET 202 that is due to at least one of pressure and physical stresses is compensated for in a more sophisticated manner than in the apparatus 200A of Figure 2A. For example, in exemplary embodiments the processing device 106 is configured to perform adaptive calibration using an embedded software application. This allows for faster and less expensive changes compared to the apparatus 200A of Figure 2A because passive components do not need to be replaced.

The output of the amplifier 216 is communicatively coupled to the processing device 106. The processing device 106 receives both outputs from the amplifier 216 and the analog-to-digital converter 302. The processing device 106 compensates the signal from the amplifier 216 using the output from the analog-to-digital converter 302. In exemplary embodiments, the compensation by the processing device 106 involves using compensation tables, compensation curves, and/or filtering. The final pH determination made by the apparatus 300A compensates for at least one of pressure and physical stresses and is more accurate than other deep sea pH sensors that do not provide digital feedback to the processing device 106.

In exemplary embodiments, the pH sensing apparatus 300A is a specific implementation of the exemplary embodiment of pH sensing apparatus 100B shown in Figure 1B and includes a pressure sensitivity compensation loop 102, an ion-sensing cell 104, and the processing device 106 as shown in Figure 3A. In exemplary embodiments, the ion-sensing cell 104 of pH sensing apparatus 300A includes the first ISFET 202, the reference electrode 214, the counter electrode 212, the amplifier 206, the amplifier 216, the voltage source 208, the resistor 301, and the voltage source 210. In exemplary embodiments, the pressure sensitivity compensation loop 102 of pH sensing apparatus 300A includes the NISFET 204, the source of potential 205, the transimpedance amplifier 222, the optional signal conditioner 224, and the analog-to-digital converter 302.

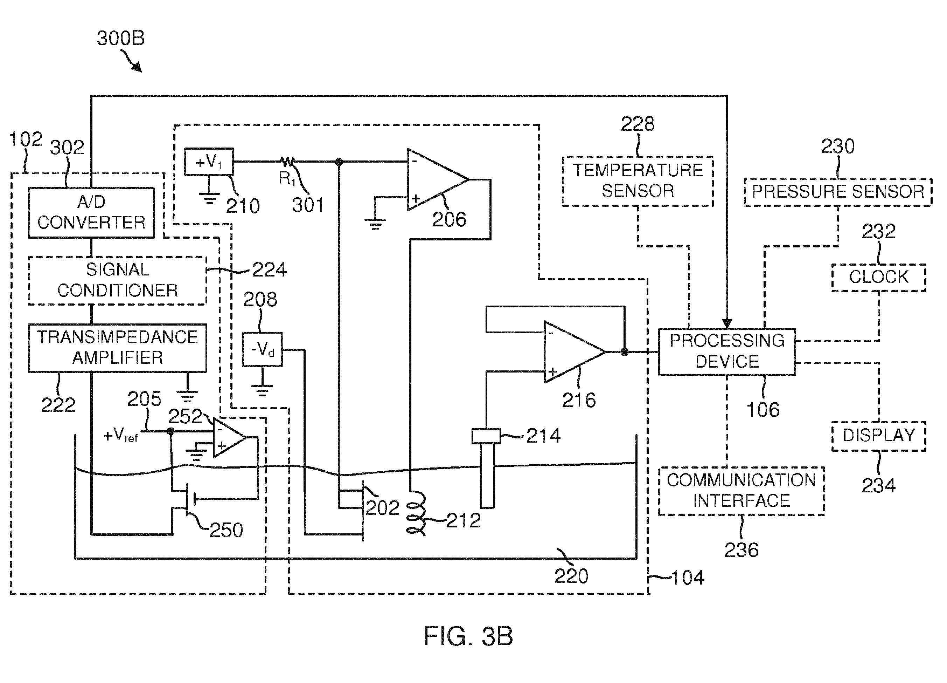

As mentioned above, in exemplary embodiments a NISFET is a sealed ISFET or a MOSFET. Figure 3B shows an embodiment of a pH sensing apparatus 300B in accordance with the present invention. The pH sensing apparatus 300B is the same as pH sensing apparatus 300A, except that the NISFET 204 of Figure 3A is replaced with a NISFET 250, which is a MOSFET. In exemplary embodiments, the size of the channel in the NISFET 250 is controlled by the output of the amplifier 252 and the source of potential 205 (+V_{ref}). The same compensation capabilities from using the differential setup of pH sensing apparatus 300A are available using the differential setup of pH sensing apparatus 300B.

In exemplary embodiments, the pH sensing apparatus 300B is a specific implementation of the exemplary embodiment of pH sensing apparatus 100B shown in Figure 1B and includes a pressure sensitivity compensation loop 102, an ion-sensing cell 104, and the processing device 106 as shown in Figure 3B. In exemplary embodiments, the ion-sensing cell 104 of pH sensing apparatus 300B includes the first ISFET 202, the reference electrode 214, the counter electrode 212, the amplifier 206, the amplifier 216, the voltage source 208, the resistor 301, and the voltage source 210. In exemplary embodiments, the pressure sensitivity compensation loop 102 of pH sensing apparatus 300A includes the NISFET 250, the source of potential 205, the amplifier 252, the transimpedance amplifier 222, the optional signal conditioner 224, and the analog-to-digital converter 302.

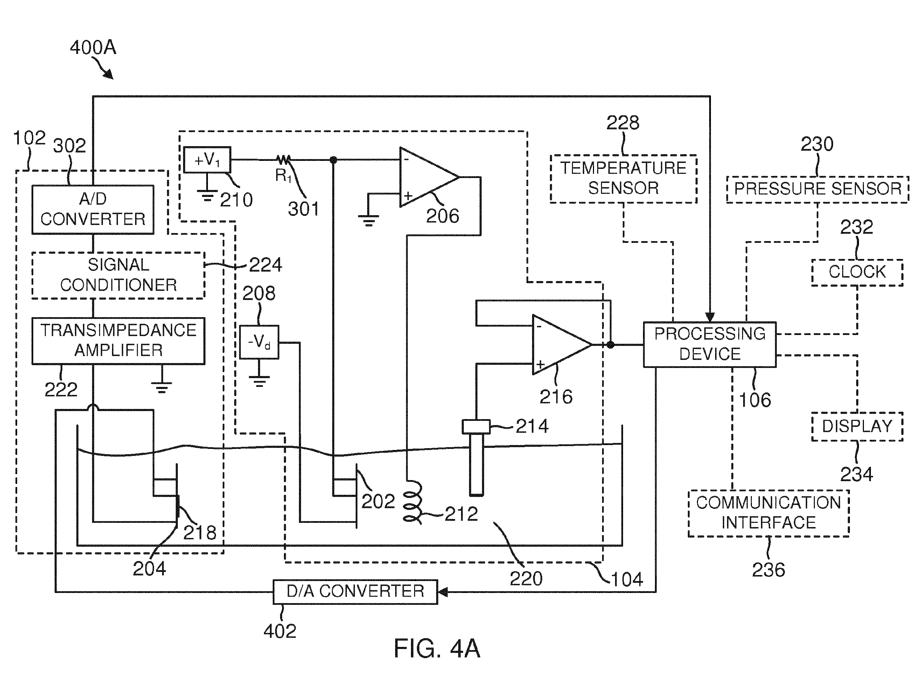

Figure 4A shows another embodiment of a pH sensing apparatus 400A in accordance with the present invention. This embodiment is similar to the apparatus 300A of Figure 3A, so only the differences in the circuit will be discussed.

Generally, the apparatus 400A includes a greater amount of digitization in the circuit compared to the apparatus 300A of Figure 3A. In the apparatus 400A, the drain current of the NISFET 204 is controlled by the processing device 106. Specifically, the processing device 106 sends a signal to the source of the NISFET 204, which is converted from a digital signal to a voltage by a digital-to-analog converter 402. By replacing the source of potential 205 (+V_{ref}) with this connection, the voltage supplied to the source of the NISFET 204 can be more easily controlled and adjusted. This greater level of control allows more sophisticated compensations to be performed.

In exemplary embodiments, the pH sensing apparatus 400A is a specific implementation of the exemplary embodiment of pH sensing apparatus 100C shown in Figure 1C and includes a pressure sensitivity compensation loop 102, an ion-sensing cell 104, and the processing device 106 as shown in Figure 4A. In exemplary embodiments, the ion-sensing cell 104 of pH sensing apparatus 400A includes the first ISFET 202, the reference electrode 214, the counter electrode 212, the amplifier 206, the amplifier 216, the voltage source 208, the resistor 301, and the voltage source 210. In exemplary embodiments, the pressure sensitivity compensation loop 102 of pH sensing apparatus 400A includes the NISFET 204, the transimpedance amplifier 222, the optional signal conditioner 224, and the analog-to-digital converter 302.

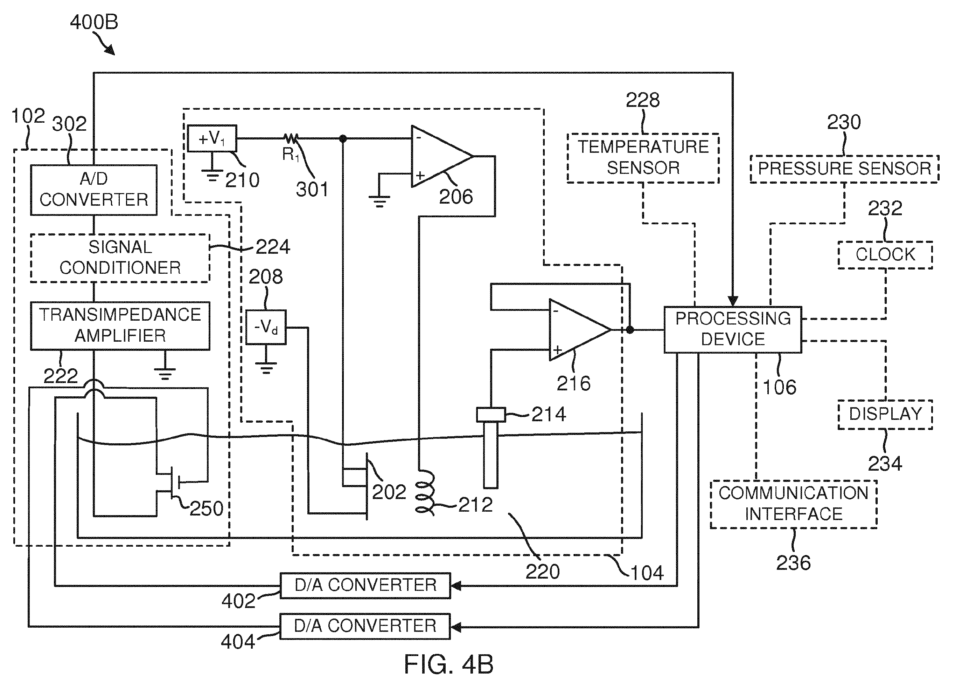

As mentioned above, in exemplary embodiments a NISFET is a sealed ISFET or a MOSFET. Figure 4B shows an embodiment of a pH sensing apparatus 400B in accordance with the present invention. The pH sensing apparatus 400B is similar to pH sensing apparatus 400A. However, there are some differences between the apparatus 400B and the apparatus 400A. In apparatus 400B, the NISFET 204 from apparatus 400A is replaced with the NISFET 250, which is a MOSFET. There is also an additional connection from the processing device 106 to the sealed gate region of the NISFET 250. The processing device 106 sends a signal to the sealed gate of the NISFET 250, which is converted from a digital signal to a voltage by a digital-to-analog converter 404. This variation from the apparatus 300B of Figure 3B allows the processing device 106 to control the size of the channel in the NISFET 250.

By allowing the processing device 106 to influence the NISFET 250, in addition to performing the compensations discussed above, the level of sophistication of the compensation increases for the apparatus 400B of Figure 4B over that of the apparatus 300B of Figure 3B. For example, common mode errors of the ISFET 202 and the NISFET 250 can be compensated for in the apparatus 400B.

In exemplary embodiments, the pH sensing apparatus 400B is a specific implementation of the exemplary embodiment of pH sensing apparatus 100C shown in Figure 1C and includes a pressure sensitivity compensation loop 102, an ion-sensing cell 104, and the processing device 106 as shown in Figure 4B. In exemplary embodiments, the ion-sensing cell 104 of pH sensing apparatus 400B includes the first ISFET 202, the reference electrode 214, the counter electrode 212, the amplifier 206, the amplifier 216, the voltage source 208, the resistor 301, and the voltage source 210. In exemplary embodiments, the pressure sensitivity compensation loop 102 of pH sensing apparatus 400B includes the NISFET 250, the transimpedance amplifier 222, the optional signal conditioner 224, and the analog-to-digital converter 302.

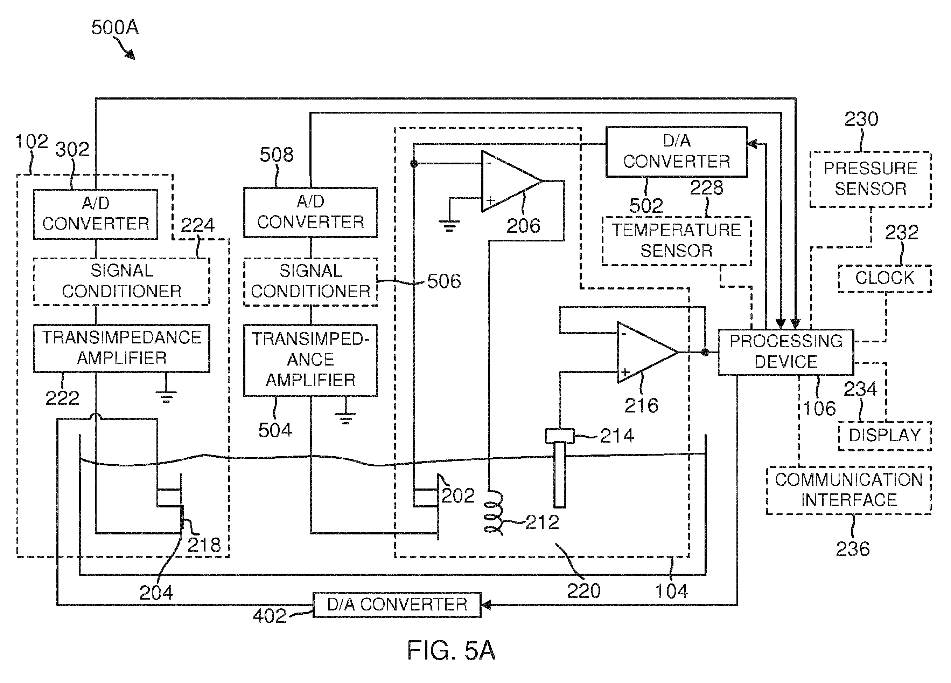

Figure 5A shows another embodiment of a pH sensing apparatus 500A in accordance with the present invention. This embodiment is similar to the apparatus 400A of Figure 4A, so only the differences in the circuit will be discussed.

Generally, the apparatus 500A includes an even greater amount of digitization in the circuit compared to the apparatus 400A of Figure 4A. In the apparatus 500A, the processing device 106 provides a voltage to the ISFET 202 and the amplifier 206. The processing device 106 sends a digital signal, which is converted to a voltage by a digital-to-analog converter 502, to the inverting input of the amplifier 206 and the source of the ISFET 202. This voltage is determined using the feedback from the NISFET 204, similar to the apparatus 200 of Figure 2. However, since the processing device 106 is sending the signal, the voltage source 210 and the resistor 301 from Figures 3-4 are not necessary in this embodiment.

The drain of the ISFET 202 is communicatively coupled to the processing device 106 and the ISFET 202 provides feedback through this connection. The signal coming from the drain of the ISFET 202 is a current. This signal is converted to a voltage by a transimpedance amplifier 504. The voltage signal generated by the transimpedance amplifier 504 can optionally be filtered by a signal conditioner 506. This signal is then converted to a digital signal by the analog-to-digital converter 508 and received by the processing device 106.

The feedback provided from the processing device 106 to the ISFET 202 and the amplifier 206 allows the processing device 106 to control the voltage that is driving these devices. In an exemplary embodiment, in addition to all the compensation previously discussed, the processing device 106 compensates for the at least one of pressure and physical stresses by trimming the voltage supplied to the ISFET 202. Also, since the NISFET 204 is providing feedback directly to the processing device 106, multiple levels of compensation may be utilized to produce a more accurate pH determination.

In exemplary embodiments, the pH sensing apparatus 500A is a specific implementation of the exemplary embodiment of pH sensing apparatus 100D shown in Figure 1D and includes a pressure sensitivity compensation loop 102, an ion-sensing cell 104, and the processing device 106 as shown in Figure 5A. In exemplary embodiments, the ion-sensing cell 104 of pH sensing apparatus 500A includes the first ISFET 202, the reference electrode 214, the counter electrode 212, the amplifier 206, and the amplifier 216. In exemplary embodiments, the pressure sensitivity compensation loop 102 of pH sensing apparatus 500A includes the NISFET 204, the transimpedance amplifier 222, the optional signal conditioner 224, and the analog-to-digital converter 302.

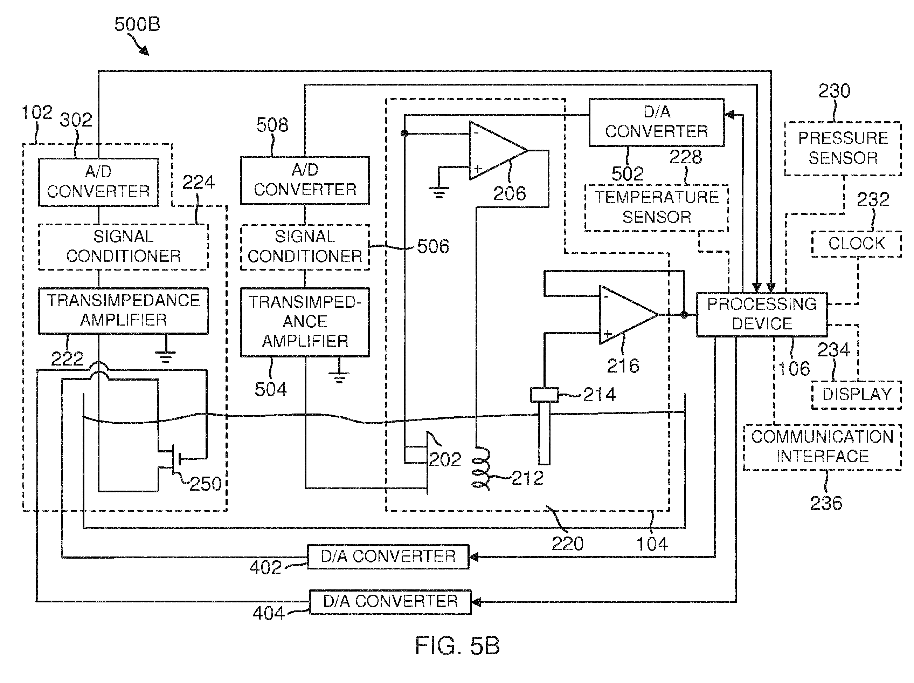

As mentioned above, in exemplary embodiments a NISFET is a sealed ISFET or a MOSFET. Figure 5B shows an embodiment of a pH sensing apparatus 500B in accordance with the present invention. The pH sensing apparatus 500B is similar to pH sensing apparatus 500A. However, there are some differences between the apparatus 500B and the apparatus 500A. In apparatus 500B, the NISFET 204 from apparatus 500A is replaced with the NISFET 250, which is a MOSFET. There is also an additional connection from the processing device 106 to the sealed gate region of the NISFET 250. The processing device 106 sends a signal to the sealed gate of the NISFET 250, which is converted from a digital signal to a voltage by a digital-to-analog converter 404. This connection allows the processing device 106 to control the size of the channel in the NISFET 250.

In exemplary embodiments, the pH sensing apparatus 500B is a specific implementation of the exemplary embodiment of pH sensing apparatus 100D shown in Figure 1D and includes a pressure sensitivity compensation loop 102, an ion-sensing cell 104, and the processing device 106 as shown in Figure 5B. In exemplary embodiments, the ion-sensing cell 104 of pH sensing apparatus 500B includes the first ISFET 202, the reference electrode 214, the counter electrode 212, the amplifier 206, and the amplifier 216. In exemplary embodiments, the pressure sensitivity compensation loop 102 of pH sensing apparatus 500B includes the NISFET 250, the transimpedance amplifier 222, the optional signal conditioner 224, and the analog-to-digital converter 302.

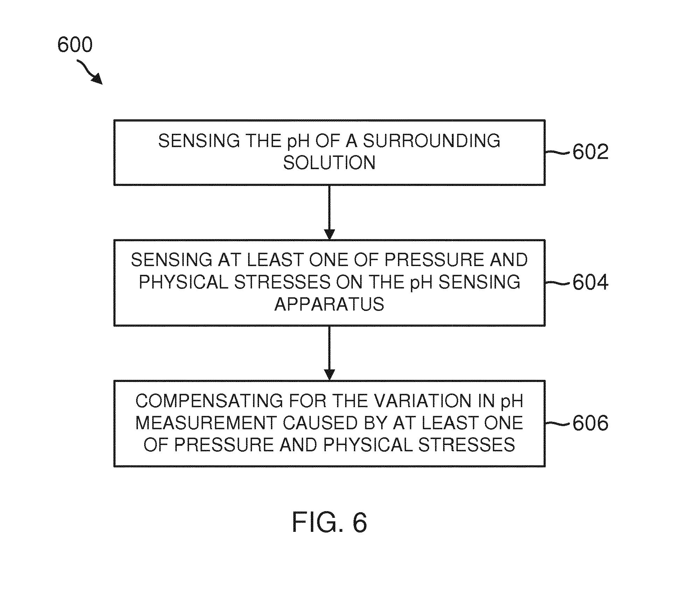

Figure 6 is a flow diagram representative of a method 600 of limiting measurement error for an output of a pH sensing apparatus in accordance with the present invention. In exemplary embodiments, the pH sensing apparatus described with reference to method 600 is any of apparatuses 100A, 100B, 100C, 100D, 200, 300, 400, or 500.

At block 602, the pH sensing apparatus is placed in a solution under test (such as solution under test 220), such as sea water. A voltage source (such as the voltage source 210) is used to power a first ISFET (such as first ISFET 202) and an amplifier (such as amplifier 206). When the apparatus is placed in the solution under test, the potential across the gate of the ISFET is affected by the ions and will result in a charge flowing through the device. The potential from the reference electrode (such as reference electrode 214) will then be measured and compared to circuit common by a processing device. A pH reading can be determined by measuring the difference between the reference electrode potential and the potential of the ISFET, which is at circuit common.

At block 604, a NISFET (such as NISFET 204 or NISFET 250) will measure the effects of at least one of pressure and physical stresses on the ISFET. Even though the NISFET is not affected by the ions of the solution under test, the NISFET will still be affected by temperature, pressure and/or physical stresses from packaging, viscoelastic or stress relaxation with time, and thermo-mechanical stresses. In exemplary embodiments, the variation of the voltage output of the NISFET will represent only the effects due to pressure and/or other physical stresses and not changes in the pH of the solution under test.

At block 606, the pH reading from block 602 is compensated for at least one of pressure and physical stresses by using the feedback from the NISFET. This step can be performed in multiple ways, which are discussed in more detail in the following paragraphs.

In one exemplary embodiment, the output from the NISFET provides feedback through an analog signal to trim the voltage source that is driving the ISFET and amplifier. This adjusts the potential of the ISFET. By making this adjustment to the voltage source, the pH sensing step of block 602 will produce a pH reading that is compensated for pressure and physical stress.

In other exemplary embodiments, the output from the NISFET provides feedback to the processing device through a digital signal. The processing device then performs a compensation of the pH measurement for errors caused by at least one of pressure and physical stresses. In exemplary embodiments, this compensation involves using compensation tables, compensation curves, and/or filtering. This does not involve sending a feedback signal to adjust the potential of the ISFET.

In another exemplary embodiment, the processing device sends a signal to the ISFET to adjust the potential of the ISFET. The processing device uses the feedback from the NISFET in order to determine what signal should be sent to the ISFET. The processing device will also adjust the potential of the ISFET as part of the calculation performed when making the final pH determination.

Processing device 106 includes or functions with software programs, firmware or other computer readable instructions for carrying out various methods, process tasks, calculations, and control functions, used in the pH sensing apparatus. These instructions are typically stored on any appropriate computer readable medium used for storage of computer readable instructions or data structures. The computer readable medium can be implemented as any available media that can be accessed by a general purpose or special purpose computer or processor, or any programmable logic device. Suitable processor-readable media may include storage or memory media such as magnetic or optical media. For example, storage or memory media may include conventional hard disks, Compact Disk - Read Only Memory (CD-ROM), volatile or non-volatile media such as Random Access Memory (RAM) (including, but not limited to, Synchronous Dynamic Random Access Memory (SDRAM), Double Data Rate (DDR) RAM, RAMBUS Dynamic RAM (RDRAM), Static RAM (SRAM), etc.), Read Only Memory (ROM), Electrically Erasable Programmable ROM (EEPROM), and flash memory, etc. Suitable processor-readable media may also include transmission media such as electrical, electromagnetic, or digital signals, conveyed via a communication medium such as a network and/or a wireless link.

Although specific embodiments have been illustrated and described herein, it will be appreciated by those of ordinary skill in the art that any arrangement, which is calculated to achieve the same purpose, may be substituted for the specific embodiments shown. Therefore, it is manifestly intended that this invention be limited only by the claims and the equivalents thereof.

### Example Embodiments

Example 1 includes a pH sensing apparatus comprising: an ion-sensing cell, wherein the ion-sensing cell includes: a first half-cell including a first Ion-Sensitive Field Effect Transistor (ISFET) exposed to a surrounding solution; and a second reference half-cell exposed to the surrounding solution; a pressure sensitivity compensation loop including a Non Ion-Sensitive Field Effect Transistor (NISFET): wherein the pH sensing apparatus is configured to compensate for at least one of pressure and physical stresses using signals from the ion-sensing cell and feedback from the pressure sensitivity compensation loop; and a processing device configured to calculate a final pH reading compensated to minimize the at least one of pressure and physical stresses.

Example 2 includes the pH sensing apparatus of Example 1, wherein the NISFET is selected from a group consisting of: a second ISFET which is sealed and non-sensitive to the ions of the surrounding solution; and a Metal-Oxide-Semiconductor Field Effect Transistor (MOSFET) which is non-sensitive to the ions of the surrounding solution.

Example 3 includes the pH sensing apparatus of any of Examples 1-2, wherein the NISFET is a second ISFET sealed with at least one of: a metal deposition selected from a group consisting of gold, platinum, titanium, tantalum, nickel, chromium, aluminum, tungsten, iridium, and silver; and an insulative deposition selected from a group consisting of silicon oxide, aluminum oxide, diamond like carbon (DLC), aluminum nitride, glass compositions, tantalum oxide, beryllium oxide, and silicon nitride.

Example 4 includes the pH sensing apparatus of any of Examples 1-3, wherein the first ISFET and the NISFET have a common silicon substrate.

Example 5 includes the pH sensing apparatus of any of Examples 1-4, wherein the first half-cell of the ion-sensing cell further comprises a counter electrode.

Example 6 includes the pH sensing apparatus of any of Examples 1-5, wherein the reference half-cell comprises at least one of: a reference electrode; and a Reference Field Effect Transistor (REFET) and a quasi-reference electrode.

Example 7 includes the pH sensing apparatus of any of Examples 1-6, wherein the pressure sensitivity compensation loop is communicatively coupled to the first half-cell of the ion-sensing cell.

Example 8 includes the pH sensing apparatus of any of Examples 1-7, wherein the pressure sensitivity compensation loop is communicatively coupled to the processing device.

Example 9 includes the pH sensing apparatus of any of Examples 1-8, wherein the processing device sends feedback to at least one of: the first ISFET; and the NISFET.

Example 10 includes the pH sensing apparatus of any of Examples 1-9, further comprising at least one of: at least one temperature sensor configured to measure the temperature at a point in the pH sensing apparatus; at least one pressure sensor configured to measure the pressure at the point in the pH sensing apparatus; at least one reference clock configured to synchronize at least one component of the pH sensing apparatus; at least one display configured to display the final pH reading; and at least one communication interface configured to communicate the compensated pH reading to at least one of another system, another device, and another apparatus.

Example 11 includes the pH sensing apparatus of any of Examples 1-10, wherein the processing device is further configured to compensate for a thermal gradient between a plurality of points in the apparatus; wherein a plurality of temperature sensors measure the temperature at the plurality of points in the apparatus; wherein the plurality of temperature sensors are synchronized by at least one reference clock such that the plurality of temperature sensors measure temperature at substantially the same time; wherein the processing device is further configured to determine the thermal gradient between the plurality of points based on a difference in temperature at the plurality of points in the apparatus and a known distance between the plurality of temperature sensors.

Example 12 includes a method of limiting measurement error for an output of a pH sensing apparatus, the method comprising: sensing the pH of a surrounding solution using an ion-sensing cell that includes a first Ion-Sensitive Field Effect Transistor (ISFET); sensing at least one of pressure and physical stresses on the pH sensing apparatus using a Non Ion-Sensitive Field Effect Transistor (NISFET); compensating for the variation in pH measurement caused by at least one of pressure and physical stresses.

Example 13 includes the method of Examples 12, wherein the NISFET is selected from a group consisting of: a second ISFET which is sealed and non-sensitive to the ions of the surrounding solution; and a Metal-Oxide-Semiconductor Field Effect Transistor (MOSFET) which is non-sensitive to the ions of the surrounding solution.

Example 14 includes the method of any of Examples 12-13, wherein the compensating is performed by sending analog feedback from the NISFET to the first ISFET.

Example 15 includes the method of any of Examples 12-14, wherein the compensating is performed by sending digital feedback from the NISFET to a processing device.

Example 16 includes the method of any of Examples 12-15, wherein the compensating is performed by sending digital feedback from the first ISFET and the NISFET to a processing device, and sending feedback from the processing device to at least one of: the first ISFET; and the NISFET.

Example 17 includes the method of any of Examples 12-16, further comprising at least one of: measuring the temperature at a point in the pH sensing apparatus; measuring the pressure at the point in the pH sensing apparatus; synchronizing at least one component of the pH sensing apparatus with at least one reference clock; displaying a final compensated pH reading with at least one display; and communicating the output of the pH sensing apparatus to at least one of another system, another device, and another apparatus.

Example 18 includes the method of any of Examples 12-17, further comprising compensating the pH measurement for a thermal gradient between a plurality of points in the apparatus by: measuring the temperature at a plurality of points in the apparatus using a plurality of temperature sensors; synchronizing the plurality of temperatures sensors using at least one reference clock such that the plurality of temperature sensors measure temperature at substantially the same time; determining the thermal gradient between the plurality of points based on a difference in temperature at the plurality of points in the apparatus and a known distance between the plurality of temperature sensors.

Example 19 includes a pH sensing apparatus comprising: an ion-sensing cell, wherein the ion-sensing cell includes: a first half-cell including: a first Ion-Sensitive Field Effect Transistor (ISFET) exposed to a surrounding solution; and a counter electrode exposed to the surrounding solution; and a second reference half-cell exposed to the surrounding solution; a pressure sensitivity compensation loop including a Non Ion-Sensitive Field Effect Transistor (NISFET); wherein the pH sensing apparatus is configured to compensate for at least one of pressure and physical stresses using signals from the ion-sensing cell and feedback from the pressure sensitivity compensation loop; a processing device configured to calculate a final pH reading compensated to minimize the at least one of pressure and physical stresses; wherein the pressure sensitivity compensation loop and the ion-sensing cell provide digital feedback to the processing device; and wherein the processing device provides feedback to the pressure sensitivity compensation loop.

Example 20 includes the pH sensing apparatus of Example 19, wherein the NISFET is selected from a group consisting of: a second ISFET which is sealed and non-sensitive to the ions of the surrounding solution; and a Metal-Oxide-Semiconductor Field Effect Transistor (MOSFET) which is non-sensitive to the ions of the surrounding solution.

Example 21 includes the pH sensing apparatus of any of Examples 19-20, wherein the processing device provides feedback to the ion-sensing cell.

Example 22 includes the pH sensing apparatus of any of Examples 19-21, wherein the NISFET is a second ISFET sealed with at least one of: a metal deposition selected from a group consisting of gold, platinum, titanium, tantalum, nickel, chromium, aluminum, tungsten, iridium, and silver; and an insulative deposition selected from a group consisting of silicon oxide, aluminum oxide, diamond like carbon (DLC), aluminum nitride, glass compositions, tantalum oxide, beryllium oxide, and silicon nitride.

Example 23 includes the pH sensing apparatus of any of Examples 19-22, wherein the first ISFET and the NISFET have a common silicon substrate.

Example 24 includes the pH sensing apparatus of any of Examples 19-23, wherein the reference half-cell comprises at least one of: a reference electrode; and a Reference Field Effect Transistor (REFET) and a quasi-reference electrode.

Example 25 includes the pH sensing apparatus of any of Examples 19-24, further comprising at least one of: at least one temperature sensor configured to measure the temperature at a point in the pH sensing apparatus; at least one pressure sensor configured to measure the pressure at the point in the pH sensing apparatus; at least one reference clock configured to synchronize at least one component of the pH sensing apparatus; at least one display configured to display the final pH reading; and at least one communication interface configured to communicate the compensated pH reading to at least one of another system, another device, and another apparatus.

Example 26 includes the pH sensing apparatus of any of Examples 19-25, wherein the processing device is further configured to compensate for a thermal gradient between a plurality of points in the apparatus; wherein a plurality of temperature sensors measure the temperature at the plurality of points in the apparatus; wherein the plurality of temperature sensors are synchronized by at least one reference clock such that the plurality of temperature sensors measure temperature at substantially the same time; wherein the processing device is further configured to determine the thermal gradient between the plurality of points based on a difference in temperature at the plurality of points in the apparatus and a known distance between the plurality of temperature sensors.

## Claims

1. A pH sensing apparatus (100A, 100B, 100C, 100D, 200A, 200B, 300A, 300B, 400A, 400B, 500A, 500B) comprising:
an ion-sensing cell (104), wherein the ion-sensing cell (104) includes:
a first half-cell (104A) including a first Ion-Sensitive Field Effect Transistor (ISFET) (202) exposed to a surrounding solution (220); and
a second reference half-cell (104B) exposed to the surrounding solution (220);
a pressure sensitivity compensation loop (102) including a Non Ion-Sensitive Field Effect Transistor (NISFET) (204, 250);
wherein the pH sensing apparatus (100A, 100B, 100C, 100D, 200A, 200B, 300A, 300B, 400A, 400B, 500A, 500B) is configured to compensate for at least one of pressure and physical stresses using signals from the ion-sensing cell (104) and feedback from the pressure sensitivity compensation loop (102); and
a processing device (106) configured to calculate a final pH reading compensated to minimize the at least one of pressure and physical stresses.

2. The pH sensing apparatus (100A, 100B, 100C, 100D, 200A, 200B, 300A, 300B, 400A, 400B, 500A, 500B) of claim 1, wherein the NISFET (204, 250) is selected from a group consisting of:
a second ISFET (204) which is sealed (218) and non-sensitive to the ions of the surrounding solution (220); and
a Metal-Oxide-Semiconductor Field Effect Transistor (MOSFET) (250) which is non-sensitive to the ions of the surrounding solution (220).

3. The pH sensing apparatus (100A, 100B, 100C, 100D, 200A, 200B, 300A, 300B, 400A, 400B, 500A, 500B) of claim 1, wherein the pressure sensitivity compensation loop (102) is communicatively coupled to the first half-cell (104A) of the ion-sensing cell (104).

4. The pH sensing apparatus (100A, 100B, 100C, 100D, 200A, 200B, 300A, 300B, 400A, 400B, 500A, 500B) of claim 1, wherein the pressure sensitivity compensation loop (102) is communicatively coupled to the processing device (106).

5. The pH sensing apparatus (100A, 100B, 100C, 100D, 200A, 200B, 300A, 300B, 400A, 400B, 500A, 500B) of claim 1, wherein the processing device (106) sends feedback to at least one of:
the first ISFET (202); and
the NISFET (250).

6. The pH sensing apparatus (100A, 100B, 100C, 100D, 200A, 200B, 300A, 300B, 400A, 400B, 500A, 500B) of claim 1, further comprising at least one of:
at least one temperature sensor (228) configured to measure the temperature at a point in the pH sensing apparatus;
at least one pressure sensor (230) configured to measure the pressure at the point in the pH sensing apparatus;
at least one reference clock (232) configured to synchronize at least one component of the pH sensing apparatus;
at least one display (234) configured to display the final pH reading; and
at least one communication interface (236) configured to communicate the compensated pH reading to at least one of another system, another device, and another apparatus.

7. The pH sensing apparatus (100A, 100B, 100C, 100D, 200A, 200B, 300A, 300B, 400A, 400B, 500A, 500B) of claim 1, wherein the processing device is further configured to compensate for a thermal gradient between a plurality of points in the apparatus;
wherein a plurality of temperature sensors (228) measure the temperature at the plurality of points in the apparatus;
wherein the plurality of temperature sensors (228) are synchronized by at least one reference clock (232) such that the plurality of temperature sensors (228) measure temperature at substantially the same time;
wherein the processing device (106) is further configured to determine the thermal gradient between the plurality of points based on a difference in temperature at the plurality of points in the apparatus and a known distance between the plurality of temperature sensors (228).

8. A method (600) of limiting measurement error for an output of a pH sensing apparatus (100A, 100B, 100C, 100D, 200A, 200B, 300A, 300B, 400A, 400B, 500A, 500B), the method comprising:
sensing (602) the pH of a surrounding solution (220) using an ion-sensing cell that includes a first Ion-Sensitive Field Effect Transistor (ISFET) (202);
sensing (604) at least one of pressure and physical stresses on the pH sensing apparatus (100A, 100B, 100C, 100D, 200A, 200B, 300A, 300B, 400A, 400B, 500A, 500B) using a Non Ion-Sensitive Field Effect Transistor (NISFET) (204, 250);
compensating (606) for the variation in pH measurement caused by at least one of pressure and physical stresses.

9. The method (600) of claim 8, wherein the NISFET (204, 250) is selected from a group consisting of:
a second ISFET (204) which is sealed (218) and non-sensitive to the ions of the surrounding solution (220); and
a Metal-Oxide-Semiconductor Field Effect Transistor (MOSFET) (250) which is non-sensitive to the ions of the surrounding solution (220).

10. The method (600) of claim 8, wherein the compensating is performed by at least one of:
sending analog feedback from the NISFET (204, 250) to the first ISFET (202);
sending digital feedback from the NISFET (205, 250) to a processing device (106); and
sending digital feedback from the first ISFET (205) and the NISFET (205, 250) to a processing device, and sending feedback from the processing device to at least one of:
the first ISFET; and
the NISFET.
